# EUROPEAN PATENT APPLICATION

(11) **EP 1 964 510 A1**
(43) Date of publication of application: **03.09.2008**
(21) Application number: 07103169.4
(22) Date of filing: 27.02.2007
(51) Int. Cl.: A61B 3/12

(54) **Imaging of phase objects**

(71) Applicant: National University of Ireland Galway, Galway (IE)
(72) Inventor: Logean, Eric, Galway (IE); Dainty, Christopher, County Galway (IE)
(74) Representative: Brophy, David Timothy

(57) **Abstract**

An apparatus for imaging phase objects located adjacent to a light-scattering surface, such as the transparent structures located in the interior of the eye adjacent to the diffusing surface of the fundus of the eye, has an illumination system which concentrates light through the eye onto a secondary point-like source on the eye fundus. Light scattered from this secondary source traverses the interior volume of the eye before exiting and being collected by an imaging system. Fourier filtering or phase contrast technique is used to image phase structures from within the eye. Adaptive optics compensate for aberrations introduced by the eye's optics thereby reducing the extent of the secondary point source and increasing the resolution of imaging provided therefrom.

## Description

This invention relates to the imaging of phase objects, i.e. objects which are translucent or transparent and which alter the phase of light passing through them. The invention has particular application in imaging the interior of the eye, but is not limited to such application.

In-vivo imaging of the eye fundus is routinely performed for diagnosis of eye diseases and during different therapeutic interventions. Conventional imaging devices such as direct/indirect ophthalmoscopes, slit-lamp microscopes, fundus cameras, and scanning laser ophthalmoscopes, image spatial variations of absorption and scattering. Translucent/transparent materials are not visible using these devices.

In the eye, all the tissues from the cornea to the photoreceptor cells are transparent to light. The retina is made up of different populations of neural cells whose task is to process the visual information and transmit it to the brain. There is an interest in in-vivo imaging of all of them. The ganglion cells and their axons are of special interest as these cells are particularly vulnerable to the degenerative disease of glaucoma.

The retinal microcirculation is affected by most ocular diseases and is of great interest to ophthalmologists. Scientists studying stroke are attracted because this circulation is easily accessible and has characteristics similar to the brain microcirculation. So far, however, only the blood column can be visualised. The study of the retinal microcirculation and its regulation could benefit from images of the vessel wall and of the layer free of red blood cells present in these vessels, both features being transparent.

The cells forming the crystalline lens and the ones forming the cornea are interesting to image as well. The former are of interest to study the evolution of the lens with age and the formation of cataracts. The latter are of interest to observe the healing process following "Lasik" surgery operations.

Different techniques have been developed to image the different transparent tissues of the eye. The most important of these is optical coherence tomography (OCT), a technique based on low coherence reflectometry that has a great depth sectioning capability and a very high sensitivity. OCT is very successful in imaging the shape of the cornea and of the crystalline lens and provides images of the different layers of the retina. Transparent cells, however, remain elusive.

Polarisation sensitive techniques are used to image and access characteristics of transparent objects. The thickness of the nerve fibre layer is currently measured using a commercially available ellipsometer (GdX, Carl Zeiss Meditec, Germany) and polarisation sensitive OCT. The first is not aimed at cellular imaging and the second suffers from the same limitation as other OCT techniques.

In microscopy, phase contrast techniques are successfully applied to image transparent objects. The idea of using such a technique in the eye is not new and has been suggested in U. S. Patent Specification No. 5,751,395 to Thall.

In Thall's device, a light source provides light rays which are focussed through an optical system into parallel alignment before they fall on the fundus of the retina, illuminating an area of the retina. The structures of the retinal fundus scatter the light rays back out of the eye into a viewing system forming an image of the retina in an image plane which is viewed by an observer.

The system proposed by Thall has not found any commercial application, and it is hypothesised that this is due to the fact that the proposed illumination system is not adequate for the proposed imaging task.

The invention provides an apparatus for imaging a phase object located adjacent to a light-scattering surface, the apparatus comprising:
(a) a light source;
(b) an illumination system for focussing light from said source towards said light-scattering surface;
(c) an imaging system for receiving light scattered from said light-scattering surface through said phase object and generating an image therefrom;
(d) a spatial filter located within the imaging system (c) to modify the optical transform of the object;
characterised in that:
said optical system is adapted to focus said light to a concentrated area on said light-scattering surface, to thereby provide a concentrated secondary light source positioned on said light-scattering surface.

By providing a concentrated secondary light source on the light scattering surface, rather than simply illuminating an area of the surface with parallel rays, the degree of spatial coherence of light scattered by the secondary source is maximised. This degree of spatial coherence of the light affects the efficiency of Fourier filtering or phase contrast imaging. Such techniques used with light having a low degree of spatial coherence add very little contrast to the image of a phase object. Used with light having a sufficient degree of spatial coherence such techniques can provide high contrast image of phase objects.

The degree of spatial coherence required for an efficient imaging system depends on the source area, the source to object distance, and to the spatial frequency content of the object. In the eye, the diffusing layer is the fundus of the eye and the object a phase structure in the interior of the eye. In such fixed geometry, minimising the extend of the secondary light source is the only mean to increase the degree of spatial coherence.

Preferably, the concentrated secondary light source is a point source.

In alternative concentrations, the concentrated secondary light source may be of a different shape, such as a line source or an annular source (i.e. an image of a slit or of an annulus projected and concentrated onto the light-scattering surface).

Preferably, the spatial filter within the imaging system is located in a plane conjugated with said secondary source.

Further preferably, the imaging system further comprises an imaging relay which can be translated to provide images conjugated with different phase objects.

It is further preferred that the imaging relay can be translated to the plane conjugated with the secondary source, to assist in focusing and calibrating the system.

Preferably, said optical system further comprises a subsystem for correcting optical aberrations for minimising the point spread function of said concentrated area and localising said concentrated area at the position of said light-scattering surface along the optical axis.

By correcting the aberrations occurring between the light source and the diffusing surface, one improves the volume concentration of light at the secondary source. This further idealises the secondary concentrated light source so that the imaging plane of the imaging system can reproduce with fidelity any phase objects traversed by the light as it exits the eye from this idealised virtual secondary source.

More preferably, said subsystem for correcting optical aberrations is an adaptive optical system.

In preferred embodiments, the adaptive optical system comprises a wavefront measurement component for measuring a wavefront of light received by said imaging system, a corrective element for altering the wavefront of light provided by said illumination system, and a control mechanism for controlling the operation of said corrective element in response to the output of said wavefront measuring component.

Alternatively, said subsystem for correcting optical aberrations is a phase plate tailored to correct the aberrations of the system under examination.

Examples of such phase plates are given in the following articles: (1) R. Navarro, E. Moreno-Barriuso, S. Bara, T. Mancebo. "Phase Plate for Wave-Aberration Compensation in the Human Eye." Opt. Lett., 2000, 25(4), 236-238; and (2) S. A. Burns, S. Marcos, A. E. Elsner, S. Bara. "Contrast Improvement of Confocal Retinal Imaging by Use of Phase-Correcting Plates." Opt. Lett., 2002, 27(6), 400-402.

Preferably, the light-scattering surface is a fundus of an eye, said phase object is a structure within the eye, and wherein said illumination system and adaptive optical subsystem are adapted to co-operate with the focussing system of the eye to generate said concentrated secondary light source with a minimised point spread function.

Preferably, the apparatus also includes a scanning subsystem for scanning said concentrated secondary light source along said light-scattering surface in co-operation with said illumination system.

In this way, a raster image may be built up by moving the secondary light source along the light-scattering surface.

In an alternative embodiment, the light source and said illumination system provide a plurality of secondary light sources on said light-scattering surface, and wherein said imaging system generates a plurality of spatially separated parallel images or a composite image from the light received from said plurality of secondary light sources.

The invention also provides a method of imaging a phase object located adjacent to a light-scattering surface, comprising the steps of:
(a) providing a light source;
(b) focussing light from said source towards said light-scattering surface;
(c) receiving light scattered from said light-scattering surface through said phase object and generating an image therefrom;
(d) modifying the optical transform of the object using a spatial filter located within the imaging system (c);
characterised in that:
said focussing step focuses said light to a concentrated area on said light-scattering surface, to thereby provide a concentrated secondary light source positioned on said light-scattering surface.

The invention will now be further illustrated by the following description of embodiments thereof, given by way of example only, with reference to the accompanying drawings, in which:
Fig. 1 is a schematic optical diagram of a first apparatus for imaging phase objects;
Fig. 2 is a schematic optical diagram of a second apparatus for imaging phase objects;
Fig. 3 is a schematic optical diagram of a third apparatus for imaging phase objects;
Fig. 4 is a schematic optical diagram of a fourth apparatus for imaging phase objects; and
Fig. 5 is a schematic optical diagram of a fifth apparatus for imaging phase objects.

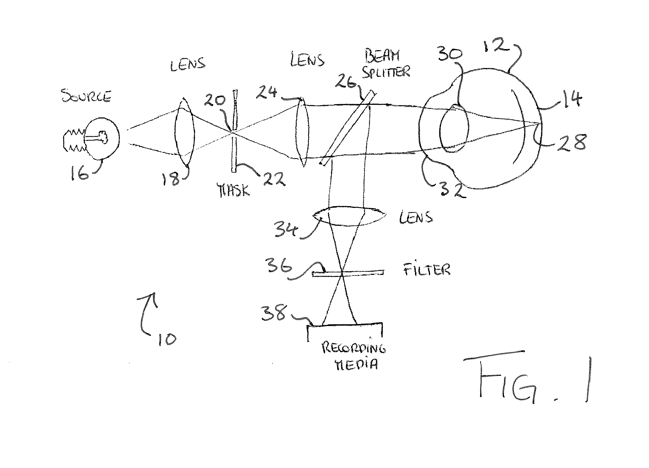

In Fig. 1 there is indicated, generally at 10, a first imaging apparatus for imaging phase objects located in the interior of an eye 12 adjacent the light-scattering surface 14 of the fundus of the eye 12.

Light from a source 16 is focussed by a lens 18 on an aperture 20 in a mask 22. A second lens 24 directs light emerging from the point source 20 of the mask 22 through a beam splitter 26 and towards the eye 12. Lens 24 is chosen and positioned to operate in conjunction with the lens and optical system of the eye 12 to focus the light to a sharp point 28 on the fundus 14 of the eye.

This focussed point of light 28 acts as a concentrated secondary light source from which light is scattered through the eye's interior volume, and light exiting through the lens 30 and cornea 32 again hits the beam splitter 26, where part of it is reflected through a third lens 34. A spatial filter 36 is conjugated with secondary point source 28. A recording medium 38 (or an optical viewing system) is positioned in the path of the light to record or view the image of the phase structures located within the interior volume of the eye, as illuminated by the secondary light source 28 created on the fundus 14.

By translating the recording medium 38 along the optical axis, different phase objects can be imaged.

The aperture of the mask 22 may be of different shape such as i) pinhole (point-source), ii) slit (line-source), and iii) annulus (annular-source). The spatial filter 36 being adapted accordingly.

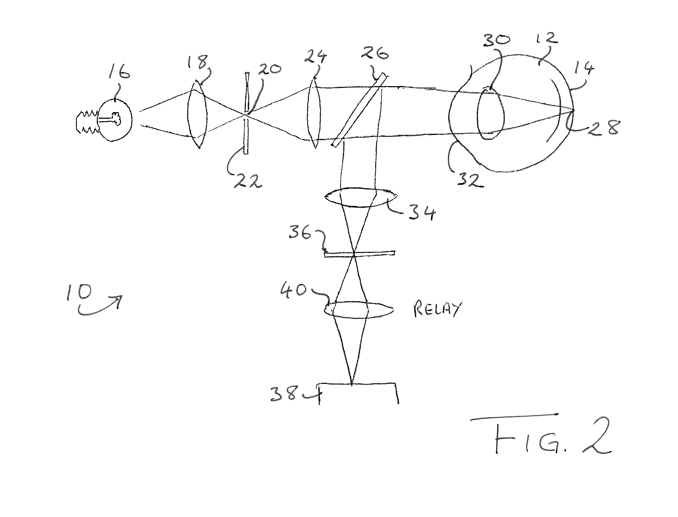

The basic system of Fig. 1 is largely reproduced in the system of Fig. 2, wherein like numerals denote like components which will not be repeatedly described. The system of Fig. 2 includes an additional optical system 40 serving as an imaging relay added after the filter plane 36. The imaged plane is selected by axially translating the relay system and the observation/recording media. Preferably, the focal range includes the plane conjugated with the secondary light source 28. This is an additional advantage that helps in aligning the system.

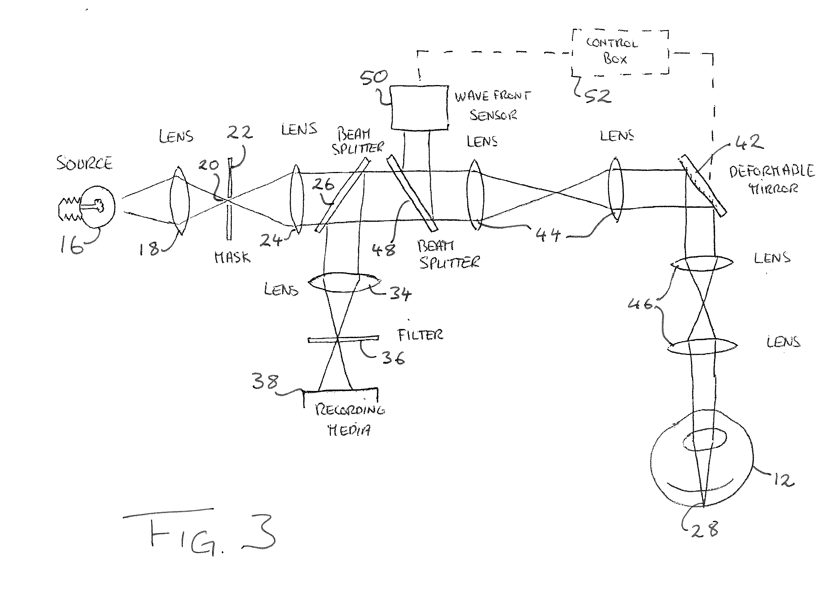

The system of Fig. 3 includes an adaptive optic system which corrects the aberrations of the eye, allowing the full aperture of the eye's pupil to be employed for retinal imaging. By increasing the aperture, the depth of field of the system can be dramatically reduced and the resolution accordingly increased. This provides a more concentrated secondary point source 28 and an imaging system with higher resolving power.

The adaptive optic system comprises a wavefront corrector 42 (here shown as a deformable mirror) which receives light from a first relay system 44 (shows as a pair of lenses) and which transmits light through a second relay system 46 to illuminate substantially the full area of the pupil of the eye 12. When light leaves the eye from secondary light source 28 and passes through relay system 46, wavefront corrector 42 and relay system 44, an additional beam splitter 48 positioned in the path of the emerging light diverts a fraction of the emerging light onto a wavefront sensor 50. This wavefront sensor detects aberrations in the wavefront, and a control box 52 of the type well known in the art operates a feedback control to deform the mirror 42 and thereby correctively adapt the wavefront of light entering the eye until the aberrations of the eye have been compensated for.

The portion of light not used for wavefront sensing is transmitted through beam splitter 26 as previously described in relation to Fig. 1.

Alternatively the adaptive optic system can use another light source. The advantage being twofold: i) all the probing light is available for imaging and ii) the intensity of each source can be balanced independently.

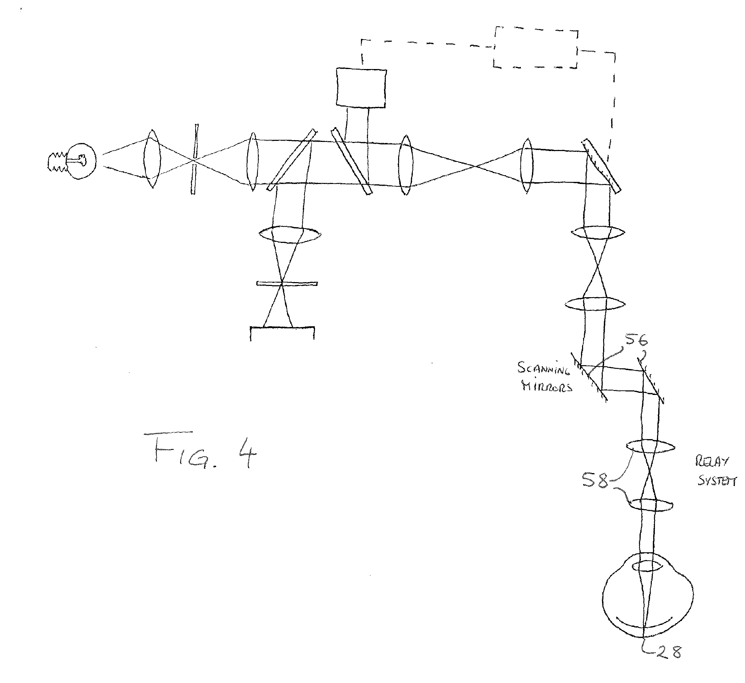

Fig. 4 shows an imaging apparatus containing all of the components of the system of Fig. 3 and in addition a scanning system comprising a pair of scanning mirrors 54 which are controllable in conjunction with an additional relay system comprising a pair of lenses 58 to move the secondary source on the retina, enabling the acquisition of a wide field image. This arrangement operates by re-imaging the plane conjugated with the pupil of the eye where the scanning mirrors are introduced. Preferably, the scanning system includes a controlling mechanism which moves the secondary source 28 in a raster fashion.

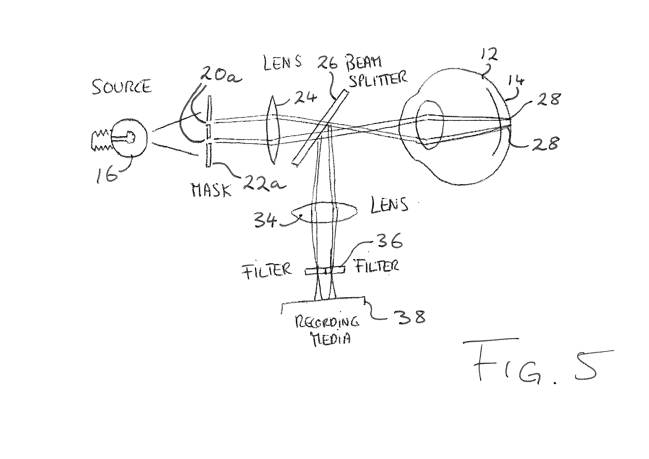

Fig. 5 illustrates the acquisition of several small field of view images acquired in parallel. The concept illustrated in Fig. 5 can be incorporated in any of the apparatuses previously described.

In Fig. 5, the mask 22a defining the source has multiple apertures 20a which define several sources (for convenience only two of these are shown). Light from these sources 20a passes through lens 24 and beam splitter 26 as previously described to illuminate the fundus 14 of the eye 12. However, these sources each illuminate a different small area of the light-scattering surface so that a plurality of secondary light sources 28 are created. Preferably, the separation between these secondary light sources is wide enough to avoid overlapping of the different parts of the object.

The light emerging from these secondary sources 28 is reflected by beam splitter 26 through lens 34 and is filtered by a spatial filter 36 arranged in the same layout as the sources. Finally, recording media 38 (or an observation system) is introduced to record in parallel the spatially separated images of the phase objects within the eye 12. Further image processing may be conducted on the multiple received images to create a composite image.

The invention is not limited to the embodiments described herein but can be amended or modified without departing from the scope of the claimed invention.

## Claims

1. An apparatus for imaging a phase object located adjacent to a light-scattering surface, the apparatus comprising:
(a) a light source;
(b) an illumination system for focussing light from said source towards said light-scattering surface;
(c) an imaging system for receiving light scattered from said light-scattering surface through said phase object and generating an image therefrom;
(d) a spatial filter located within the imaging system (c) to modify the optical transform of the object;
**characterised in that**:
said optical system is adapted to focus said light to a concentrated area on said light-scattering surface, to thereby provide a concentrated secondary light source positioned on said light-scattering surface.

2. An apparatus as claimed in claim 1, wherein said concentrated secondary light source is a point source.

3. An apparatus as claimed in claim 1, wherein said concentrated secondary light source is a line source.

4. An apparatus as claimed in claim 1, wherein said concentrated secondary light source is an annular source.

5. An apparatus as claimed in any preceding claim, wherein said imaging system further comprises an imaging relay which can be translated to provide images conjugated with different phase objects.

6. An apparatus as claimed in any preceding claim, wherein said optical system further comprises a subsystem for correcting optical aberrations for minimising the point spread function of said concentrated area and localising said concentrated area at the position of said light-scattering surface along the optical axis.

7. An apparatus as claimed in claim 6, wherein said subsystem for correcting optical aberrations is an adaptive optical system.

8. An apparatus as claimed in claim 7, wherein said adaptive optical system comprises a wavefront measurement component for measuring a wavefront of light received by said imaging system, a corrective element for altering the wavefront of light provided by said illumination system, and a control mechanism for controlling the operation of said corrective element in response to the output of said wavefront measuring component.

9. An apparatus as claimed in claim 6, wherein said subsystem for correcting optical aberrations is a phase plate tailored to correct said aberrations.

10. An apparatus as claimed in any preceding claim wherein said light-scattering surface is a fundus of an eye, said phase object is a structure within the eye, and wherein said illumination system and optical system are adapted to co-operate with the focussing system of the eye to generate said concentrated secondary light source with a minimised point spread function.

11. An apparatus as claimed in any preceding claim, further comprising a scanning subsystem for scanning said concentrated secondary light source along said light-scattering surface in co-operation with said illumination system.

12. An apparatus as claimed in any preceding claim, wherein said light source and said illumination system provide a plurality of secondary light sources on said light-scattering surface, and wherein said imaging system generates a plurality of spatially separated images or a composite image from the light received from said plurality of secondary light sources.

13. A method of imaging a phase object located adjacent to a light-scattering surface, comprising the steps of:
(a) providing a light source;
(b) focussing light from said source towards said light-scattering surface;
(c) receiving light scattered from said light-scattering surface through said phase object and generating an image therefrom;
(d) modifying the optical transform of the object using a spatial filter located within the imaging system (c);
**characterised in that**:
said focussing step focuses said light to a concentrated area on said light-scattering surface, to thereby provide a concentrated secondary light source positioned on said light-scattering surface.
